# EUROPEAN PATENT SPECIFICATION

(11) **EP 4 257 035 B1**
(45) Date of publication and mention of the grant of the patent: **07.10.2026**
(21) Application number: 23188446.1
(22) Date of filing: 19.12.2019
(51) Int. Cl.: A61F 5/445, A61B 5/00, A61B 5/103

(54) **OSTOMY CONDITION CLASSIFICATION WITH MASKING, DEVICES AND RELATED METHODS**
OSTOMIEZUSTANDSKLASSIFIZIERUNG MIT MASKIERUNG, VORRICHTUNGEN UND ZUGEHÖRIGE VERFAHREN
CLASSIFICATION D'ÉTAT DE STOMIE AVEC MASQUAGE, DISPOSITIFS ET PROCÉDÉS ASSOCIÉS

(30) Priority: 20.12.2018 DK PA201870831
(43) Date of publication of application: 11.10.2023
(62) Document number(s) of the earlier application(s) in accordance with Art. 76 EPC: 19827621.4 / 3 897 481
(73) Proprietor: Coloplast A/S, 3050 Humlebaek (DK)
(72) Inventor: Andersen, Niels Kristian Mäkinen, 2600 Glostrup (DK)

(56) References cited:
- WO-A2-2007/133555
- US-A1- 2012 253 224
- US-A1- 2014 236 111
- US-A1- 2015 150 457
- US-A1- 2017 079 576

## Description

The disclosure relates to methods and devices for classification of an ostomy condition, and in particular for image-based classification of an ostomy condition.

### Background

US2015/150457 A1 relates to a method and system for wound assessment and management. US 2017/0079576 A1 relates to a method and device for capturing and digitally storing images of a wound, fistula or stoma site.

### Brief Description of the Drawings

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Fig. 1 illustrates an exemplary method according to the disclosure
Fig. 2 illustrates an ostomy appliance and an accessory device
Fig. 3 is an appliance image of appliance image data
Fig. 4 is an appliance image of transformed appliance image data
Fig. 5 is an appliance image of appliance image data
Fig. 6 is a block diagram of an exemplary accessory device
Fig. 7 is a block diagram of an exemplary server device
Fig. 8 shows an exemplary first ostomy representation
Fig. 9 shows exemplary appliance image representations
Fig. 10 shows an exemplary second ostomy representation
Fig. 11 shows exemplary ostomy representations

### Detailed Description

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

Throughout the present disclosure, the words "stoma" and "ostomy" are used to denote a surgically created opening bypassing the intestines or urinary tract system of a person. The words are used interchangeably, and no differentiated meaning is intended. The same applies for any words or phrases derived from these, e.g. "stomal", "ostomies" etc. Also, the solid and liquid wastes emanating from the stoma may be referred to as both stomal "output," "waste(s)," and "fluids" interchangeably. A subject having undergone ostomy surgery may be referred to as "ostomist" or "ostomate" - moreover, also as "patient" or "user". However, in some cases "user" may also relate or refer to a health care professional (HCP), such as a surgeon or an ostomy care nurse or others. In those cases, it will either be explicitly stated, or be implicit from the context that the "user" is not the "patient" him- or herself.

Throughout the present disclosure, the term "stomal area" denotes the stoma and an area around the stoma (peristomal area). The "peristomal area" denotes the area around the stoma covered by the adhesive surface when the ostomy appliance is attached to the skin of the user in its intended position during use.

In the following, whenever referring to proximal side or surface of a layer, an element, a device or part of a device, the referral is to the skin-facing side or surface, when a user wears the ostomy appliance. Likewise, whenever referring to the distal side or surface of a layer, an element, a device or part of a device, the referral is to the side or surface facing away from the skin, when a user wears the ostomy appliance. In other words, the proximal side or surface is the side or surface closest to the user, when the appliance is fitted on a user and the distal side is the opposite side or surface - the side or surface furthest away from the user in use.

The axial direction is defined as the direction of the stoma, when a user wears the appliance. Thus, the axial direction is generally perpendicular to the skin or abdominal surface of the user.

A radial direction is defined as perpendicular to the axial direction. In some sentences, the words "inner" and "outer" may be used. These qualifiers should generally be perceived with respect to the radial direction, such that a reference to an "outer" element means that the element is farther away from a centre portion of the ostomy appliance than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do no provide the effect.

The present disclosure relates to methods, devices, ostomy system, and devices thereof and in particular methods and devices for classifying an ostomy condition. The ostomy system comprises one or more of an ostomy appliance and one or more accessory devices. An accessory device (also referred to as an external device) may be a mobile phone (e.g. a smartphone), tablet computer, or other handheld device. An accessory device may be a personal electronic device, e.g. a wearable, such as a watch or other wrist-worn electronic device. The ostomy system may comprise a server device. The server device may be operated and/or controlled by the ostomy appliance manufacturer and/or a service centre.

A method for classifying an ostomy condition is provided, the method comprising obtaining image data, e.g. with an accessory device, the image data comprising stoma image data of a stomal area including a stoma and/or appliance image data of an adhesive surface of an ostomy appliance; determining one or more ostomy representations including a first ostomy parameter, based on the image data, such as based on the stoma image data and/or the appliance image data, and/or based on transformed image data; and outputting the first ostomy parameter. The method further comprises transforming the image data, determining one or more image representations based on the transformed image data, and determining one or more ostomy representations including a first ostomy parameter based on the one or more image representations. The first ostomy parameter is a discoloration index indicative of discoloration of the stomal area or a leakage parameter indicative of output distribution on the adhesive surface.

The one or more ostomy representations may further include a first ostomy representation, and the method may further comprise outputting the first ostomy representation.

It is an advantage of the present disclosure that improved classification of ostomy condition is provided by compensating for image data of poor quality, e.g. image data obtained from different positions. Further, an improved classification of ostomy condition is provided by securing a uniform handling and/or interpretation of image data.

Also, it is an important advantage of the present disclosure that a more accurate classification of ostomy condition is provided by determining ostomy parameters in a uniform way. Further, the present disclosure allows for improved resolution in classifying an ostomy condition, e.g. resulting in classifying an ostomy condition into a larger number of ostomy condition types.

One or more exemplary methods for classifying an ostomy condition comprises:
obtaining image data, the image data comprising stoma image data of a stomal area including a stoma and/or appliance image data of an adhesive surface of an ostomy appliance;
determining one or more image representations based on the image data;
determining one or more ostomy representations including a first ostomy parameter based on the one or more image representations; and
outputting the first ostomy parameter and/or one or more ostomy representations.

One or more exemplary methods for classifying an ostomy condition comprises:
obtaining image data, the image data comprising stoma image data of a stomal area including a stoma and/or appliance image data of an adhesive surface of an ostomy appliance;
determining one or more ostomy representations including a first ostomy parameter, based on the image data; and
outputting the first ostomy parameter and/or one or more ostomy representations, wherein the method comprises transforming the image data, and wherein determining the one or more ostomy representations based on the image data comprises determining the first ostomy parameter based on the transformed image data.

The method comprises obtaining image data, such as stoma image data and/or appliance image data. In one or more exemplary methods, obtaining image data may comprise capturing image data with a camera and transmitting the image data to a server device. In one or more exemplary methods, obtaining image data may comprise receiving, at a server device, the image data.

The method comprises transforming the image data. In one or more exemplary methods, transforming the image data comprises transforming the image data with server device or with accessory device. Transforming the image data may comprise transmitting the transformed image data or parts thereof to server device. Transforming the image data may comprise receiving, in server device, the transformed image data or parts thereof.

The method comprises determining one or more ostomy representations, e.g. with accessory device and/or server device. Determining one or more ostomy representations may comprise receiving, with the accessory device, the one or more ostomy representations from server device.

In one or more exemplary methods, determining one or more ostomy representations including a first ostomy parameter based on the image data comprises:
determining one or more image representations based on the image data; and determining one or more ostomy representations including a first ostomy parameter based on the one or more image representations.

In one or more exemplary methods, determining one or more ostomy representations including a first ostomy parameter based on the image data comprises:
transforming the image data;
determining one or more image representations based on the transformed image data; and
determining one or more ostomy representations including a first ostomy parameter based on the one or more image representations.

An image representation may be a binary mask. Accordingly, the one or more image representations may be a binary mask. In other words, determining one or more ostomy representations may comprise determining one or more binary masks based on the image data or the transformed image data. In one or more exemplary methods, determining one or more image representations based on the image data are performed by convolutional neural network with N layers, e.g. in the range from 10-50 layers.

The method may comprise storing the one or more image representations, optionally including a stoma identifier and/or a user identifier.

In one or more exemplary methods and/or devices, the one or more image representations comprises a stoma background image representation, e.g. based on stoma image data and/or transformed stoma image data. The stoma background image representation is indicative of a background of the stoma image data, i.e. which part(s)/pixels of the stoma image data that are regarded or identified as background (e.g. including part of user skin not covered by adhesive surface of ostomy appliance). Determining one or more ostomy representations, such as the first ostomy representation and/or a third ostomy representation, may be based on the stoma background image representation. The stoma background image representation may have a resolution of 256x256 pixels or more, such as 512x512 pixels.

In one or more exemplary methods and/or devices, the one or more image representations comprises an appliance background image representation, e.g. based on appliance image data and/or transformed appliance image data. The appliance background image representation is indicative of a background of the appliance image data, i.e. which part(s)/pixels of the appliance image data that are regarded or identified as background (e.g. image part(s)/pixel(s) outside the area of the adhesive surface of htre ostomy appliance). Determining one or more ostomy representations, such as the second ostomy representation and/or a third ostomy representation, may be based on the appliance background image representation.

In one or more exemplary methods and/or devices, the one or more image representations comprises a stoma image representation, e.g. based on stoma image data and/or transformed stoma image data. The stoma image representation is indicative of the stoma, i.e. which part(s)/pixels of the ostomy image data that are regarded or identified as the stoma. Determining one or more ostomy representations, such as the first ostomy representation and/or a third ostomy representation, may be based on the stoma image representation. The stoma image representation may have a resolution of 256x256 pixels or more, such as 512x512 pixels.

In one or more exemplary methods and/or devices, the one or more image representations comprises a normal skin image representation, e.g. based on stoma image data and/or transformed stoma image data. The normal skin image representation is indicative of the normal skin of the peristomal area, i.e. which part(s)/pixels of the ostomy image data that are regarded or identified as not having discoloration. Determining one or more ostomy representations, such as the first ostomy representation and/or a third ostomy representation, may be based on the normal skin image representation. The normal skin image representation may have a resolution of 256x256 pixels or more, such as 512x512 pixels.

In one or more exemplary methods and/or devices, the one or more image representations comprises one or more, such as two, three, four or more, stoma discoloration representations. A stoma discoloration representation may be indicative of a discoloration of the peristomal area, i.e. which part(s)/pixels of the ostomy image data that are regarded or identified as the peristomal area and discoloured. A stoma discoloration representation may have a resolution of 256x256 pixels or more, such as 512x512 pixels.

In one or more exemplary methods and/or devices, the one or more image representations comprises a first stoma discoloration representation, e.g. based on stoma image data and/or transformed stoma image data. The first stoma discoloration representation may be indicative of a first discoloration of the peristomal area, i.e. which part(s)/pixels of the ostomy image data that are regarded or identified as the peristomal area and have a first discoloration (e.g. first degree of redness). The first stoma discoloration representation may be indicative of part(s)/pixels of the ostomy image data within the peristomal area having a color parameter, such as the red channel of an RGB image, within a first range or less than a first threshold, such as less than 0.25. The first stoma discoloration representation may be indicative of part(s)/pixels of the peristomal area with little or no discoloration. Determining one or more ostomy representations may be based on the first stoma discoloration representation.

In one or more exemplary methods and/or devices, the method comprises determining the first stoma discoloration representation based on red channel data of the image data/stoma image data.

In one or more exemplary methods and/or devices, the one or more image representations comprises a second stoma discoloration representation, e.g. based on stoma image data and/or transformed stoma image data. The second stoma discoloration representation may be indicative of a second discoloration of the peristomal area, i.e. which part(s)/pixels of the ostomy image data that are regarded or identified as the peristomal area and have a second discoloration (e.g. second degree of redness). The second discoloration is different from the first discoloration. The second stoma discoloration representation may be indicative of part(s)/pixels of the ostomy image data within the peristomal area having a color parameter, such as the red channel of an RGB image, within a second range, e.g. in the range from 0.25 to 0.5. The second stoma discoloration representation may be indicative of part(s)/pixels of the peristomal area with small, medium or high discoloration depending on the values of the second range. Determining one or more ostomy representations may be based on the second stoma discoloration representation.

In one or more exemplary methods and/or devices, the method comprises determining the second stoma discoloration representation based on red channel data of the image data/stoma image data.

In one or more exemplary methods and/or devices, the one or more image representations comprises a third stoma discoloration representation, e.g. based on stoma image data and/or transformed stoma image data. The third stoma discoloration representation may be indicative of a third discoloration of the peristomal area, i.e. which part(s)/pixels of the ostomy image data that are regarded or identified as the peristomal area and have a third discoloration (e.g. third degree of redness). The third stoma discoloration representation may be indicative of part(s)/pixels of the ostomy image data within the peristomal area having a color parameter, such as the red channel of an RGB image, within a third range, e.g. in the range from 0.5 to 0.75. The third stoma discoloration representation may be indicative of part(s)/pixels of the peristomal area with medium or high discoloration. Determining one or more ostomy representations may be based on the third stoma discoloration representation.

In one or more exemplary methods and/or devices, the method comprises determining the third stoma discoloration representation based on red channel data of the image data/stoma image data.

In one or more exemplary methods and/or devices, the one or more image representations comprises a fourth stoma discoloration representation, e.g. based on stoma image data and/or transformed stoma image data. The fourth stoma discoloration representation may be indicative of a fourth discoloration of the peristomal area, i.e. which part(s)/pixels of the ostomy image data that are regarded or identified as the peristomal area and have a fourth discoloration (e.g. fourth degree of redness). The fourth stoma discoloration representation may be indicative of part(s)/pixels of the ostomy image data within the peristomal area having a color parameter, such as the red channel of an RGB image, within a fourth range, e.g. in the range from 0.75 to 1, or larger than a fourth threshold. The fourth stoma discoloration representation may be indicative of part(s)/pixels of the peristomal area with high discoloration. Determining one or more ostomy representations may be based on the fourth stoma discoloration representation.

In one or more exemplary methods, the method comprises determining the fourth stoma discoloration representation based on red channel data of the image data/stoma image data.

In one or more exemplary methods, determining one or more image representations based on the image data comprises determining a base color parameter, e.g. including a first base color parameter and/or a second base color parameter, and determining the one or more image representations and/or one or more ostomy parameters based on the base color parameter. The base color parameter may be based on red channel data of the ostomy image data.

In one or more exemplary methods, determining one or more image representations and/or transforming image data optionally comprises applying an image conversion to the image data, such as the stoma image data. The image conversion may be based on one or more color channels including the red channel R and optionally the blue channel and/or the green channel of the image being converted. The converted image I_C may be given as: $I_C = Abs \left(R-Average\left(G-B\right),\right.$ where R is the red channel in the image, G is the green channel and B is the blue channel. In other words, the red, blue, and green channels of the image may be converted into a single combined channel also denoted CC for each pixel of the image. The converted image I_C may be a linear combination of the red, blue, and green channels.

In one or more exemplary methods, determining second ostomy parameter(s) may be based on the first base color parameter and/or the second base color parameter.

In one or more exemplary methods, a first base color parameter is indicative of a lower discoloration limit (i.e. corresponding to a discoloration of 0%) and optionally corresponding to a minimum of discoloration of pixels in the fourth stoma image representation (a first discoloration representation indicative of a discoloration of the peristomal area). The first base color parameter may correspond to an R or CC pixel value of 0 or the lowest R or CC pixel value in the (converted) image. The first base color parameter may be based on the CC value of pixels in the stoma image data identified as normal skin, e.g. for pixels near and outside a first boundary line indicative of a circumference or edge of the stomal area. Thus, the color of skin not being covered by adhesive may be used as a reference or baseline for no discoloration.

In one or more exemplary methods, a second base color parameter is indicative of an upper discoloration limit (i.e. corresponding to a pixel discoloration of 100%) and optionally corresponding to a maximum red channel pixel value or a maximum combined channel pixel value in the second stoma image representation (stoma image representation indicative of the stoma). Thus, the color of the stoma (which is always red) may be used as a reference color, in turn providing more uniform results and accommodating differences in light conditions when obtaining image data.

In one or more exemplary methods and/or devices, the one or more image representations comprises one or more, such as two, three, four or more, appliance discoloration representations. An appliance discoloration representation may be indicative of a discoloration of the adhesive surface of the ostomy appliance, i.e. which part(s)/pixels of the appliance image data that are regarded or identified as the adhesive surface and discoloured. An appliance discoloration representation may have a resolution of 256x256 pixels or more, such as 512x512 pixels.

The one or more image representations may comprise a first appliance discoloration representation, e.g. based on appliance image data and/or transformed appliance image data. The first appliance discoloration representation may be indicative of a first discoloration of the adhesive surface of the ostomy appliance, i.e. which part(s)/pixels of the appliance image data that are regarded or identified as the adhesive surface and have a first discoloration (e.g. first degree of output or simply output). The first appliance discoloration representation may be indicative of part(s)/pixels of the appliance image data having a color parameter, such as the red channel and/or the green channel of an RGB image, within a first range or less than a first threshold, such as less than 0.25. The first appliance discoloration representation may be indicative of part(s)/pixels of the adhesive surface with little or medium discoloration. Determining one or more ostomy representations may be based on the first appliance discoloration representation.

The one or more image representations may comprise a second appliance discoloration representation, e.g. based on appliance image data and/or transformed appliance image data. The second appliance discoloration representation may be indicative of a second discoloration of the adhesive surface of the ostomy appliance, i.e. which part(s)/pixels of the appliance image data that are regarded or identified as the adhesive surface and have a second discoloration (e.g. second degree of output). The second appliance discoloration representation may be indicative of part(s)/pixels of the appliance image data having a color parameter, such as the red channel and/or the green channel of an RGB image, within a second range or larger than a second threshold. The second appliance discoloration representation may be indicative of part(s)/pixels of the adhesive surface with medium or high discoloration. Determining one or more ostomy representations may be based on the second appliance discoloration representation.

In one or more exemplary methods and/or devices, the one or more image representations comprises a stomal opening image representation, e.g. based on appliance image data and/or transformed appliance image data. The stomal opening image representation is indicative of the stomal opening, i.e. which part(s)/pixels of the appliance image data that are regarded or identified as the stomal opening. Determining one or more ostomy representations may be based on the stomal opening image representation. The stomal opening image representation may have a resolution of 256x256 pixels or more, such as 512x512 pixels.

The one or more image representations may comprise an appliance area representation, e.g. based on appliance image data and/or transformed appliance image data. The appliance area representation may be indicative of no appliance discoloration on the adhesive surface, i.e. no leak of output and thus which part(s)/pixels of the appliance image data that are regarded or identified as the adhesive surface and not being discoloured by output. Determining one or more ostomy representations may be based on the appliance area representation.

In one or more exemplary methods, determining one or more ostomy representations comprises determining an ostomy representation by combining a plurality of image representations. Determining one or more ostomy representations may comprise overlaying one or more image representations, such as one or more stoma discoloration representations, on the stoma image data or on the transformed stoma image data. Determining one or more ostomy representations may comprise overlaying one or more image representations, such as one or more appliance discoloration representations, on the appliance image data or on the transformed appliance image data.

Determining one or more ostomy representations, such as the first ostomy representation may comprise determining a discoloration map based on the stoma image data or on the transformed stoma image data and overlaying the discoloration map on the stoma image data or on the transformed stoma image data. In other words, the first ostomy representation may comprise a discoloration map.

Determining a discoloration map based on the stoma image data or on the transformed stoma image data may comprise assigning a first color value to pixels of the peristomal area that are discoloured to a first degree in a first range. Determining a discoloration map based on the stoma image data or on the transformed stoma image data may comprise assigning a second color value to pixels of the peristomal area that are discoloured to a second degree in a second range and/or assigning a third color value to pixels of the peristomal area that are discoloured to a third degree in a third range. Four, five, six, seven, nine, ten, or more different color values may be assigned to four, five, six, seven, nine, ten, or more different ranges. Thus, the discoloration map may comprise first pixels having a first color value, second pixels having a second color value and optionally third pixels having a third color value.

In one or more exemplary methods, determining one or more ostomy representations comprises determining a first ostomy representation and/or a second ostomy representation by combining a plurality of image representations.

The method comprises outputting one or more ostomy representations, e.g. the first ostomy representation and/or the second ostomy representation, and/or outputting the first ostomy parameter. Outputting first ostomy representation and/or first ostomy parameter may comprise displaying the first ostomy parameter or a first ostomy representation comprising the first ostomy parameter on a display of an accessory device. Outputting first ostomy representation and/or first ostomy parameter may comprise receiving, in the accessory device, the first ostomy representation and/or the first ostomy parameter. Outputting first ostomy representation and/or first ostomy parameter may comprise transmitting, with server device, the first ostomy representation and/or the first ostomy parameter, e.g. to the accessory device. Outputting ostomy representation(s) may comprise storing the ostomy representations in memory of the accessory device and/or server device.

Determining the one or more ostomy representations based on the image data may comprise determining a first ostomy representation, OR_1, based on the image data, ID, and/or transformed image data, ID_T, e.g. the stoma image data and/or the appliance image data. The first ostomy representation OR_1, also optionally denoted stoma representation, may be indicative of discoloration of the stomal area of the user. The first ostomy representation OR_1 may comprise or be overlaid on the stoma image data or transformed stoma image data. The first ostomy representation may comprise the first ostomy parameter, OP_1, and/or second ostomy parameter(s), P_2_1, P_2_2, .... The first ostomy representation may comprise stoma image data, SID, and/or transformed stoma image data, SID_T.

The method comprises determining one or more ostomy representations including a first ostomy parameter based on the one or more image representations.

The first ostomy parameter may be a discoloration index indicative of discoloration of the stomal area. The second ostomy parameter, also denoted OP_2, or second set of second ostomy parameters may be indicative of discoloration of the stomal area, such as indicative of discoloration severity percentage or degrees of discoloration of the peristomal area. The second set of ostomy parameters optionally comprises two or more second parameters, such as three, four, five, six, seven, eight, nine, ten or more second parameters. The second set of ostomy parameters optionally comprises a second primary ostomy parameter, also denoted OP_2_1, and a second secondary ostomy parameter, also denoted OP_2_2. The second set of ostomy parameters optionally comprises a second tertiary ostomy parameter, also denoted OP_2_3, and/or a second quaternary ostomy parameter, also denoted OP_2_4.

The first ostomy parameter may be indicative of how much of the peristomal area that is discoloured. For example, the first ostomy parameter OP_1 may be based on the one or more stoma discoloration representations and may be given by $OP_ 1 = N_TOT / N_PA ,$

Where N_TOT is the total number of discoloured pixels in the peristomal area and N_PA is the total number of pixels in the peristomal area.

The first ostomy parameter may be indicative of an area of discoloured peristomal area.

For example, the first ostomy parameter OP_1 may be based on the one or more stoma discoloration representations and may be given by $OP_ 1 = APP * N_TOT$

Where N_TOT is the total number of discoloured pixels in the peristomal area and APP is an area per pixel.

The area of a pixel APP may be given as: $AAP = HPP * WPP ,$ wherein HPP is the height per pixel and WPP is the width per pixel.

The height per pixel HPP may be based on one or more of the image representations, such as a stoma background image representation and/or an appliance background image representation.

The height per pixel, HPP may be given as: $HPP = AH / PH ,$ wherein AH is a height of the appliance (e.g. retrieved from a database) and PH is a pixel height of the appliance optionally determined as a number of pixels between two edges of the appliance counted along a vertical axis, e.g. in the appliance background image representation.

The width per pixel, WPP may be given as $WPP = AW / PW ,$ wherein AW is a width of the appliance (e.g. retrieved from a database) and PW is a pixel width of the appliance optionally determined as the number of pixels between two edges of the appliance counted along a horizontal axis, e.g. in the appliance background image representation.

The method optionally comprises determining one or more second ostomy parameters based on the one or more image representations. The method optionally comprises outputting the one or more third second parameters.

A second primary ostomy parameter may be indicative of how many of the discoloured pixels in the peristomal area that are discoloured to a first degree or indicative of the area of pixels in the peristomal area that are discoloured to a first degree. For example, the second primary ostomy parameter OP_2_1 may be based on the one or more stoma discoloration representations and may be given by $OP_ 2 _ 1 = N_DIS_ 1 / N_TOT ,$ where N_DIS_1 is the number of discoloured pixels in the peristomal area that are discoloured to a first degree, e.g. less than 0.25, or to a first degree within a first range, where a discoloration of 0 % corresponds to a first base color parameter indicative of a lower discoloration limit of the stoma and a discoloration of 100% corresponds to a second base color parameter indicative of a maximum red channel pixel value or a maximum combined channel pixel value in the second stoma image representation (stoma image representation indicative of the stoma). N_TOT is the total number of discoloured pixels in the peristomal area. In other words, the red channel pixel intensity of each pixel in the peristomal area is evaluated and compared to a discoloration scale where 0% corresponds to a minimum of discoloration of pixels in the fourth stoma image representation (a first discoloration representation indicative of a discoloration of the peristomal area) and where 100% corresponds to a maximum red channel pixel value in the second stoma image representation (stoma image representation indicative of the stoma). Put in another way, each pixel of the fourth stoma image representation is evaluated to assign a discoloration degree (selected from at least a first degree and a second degree) to each pixel of the in a plurality of discoloration degrees.

One or more second primary ostomy parameters may be indicative of the area, degree, or number of discoloured pixels within a first region of the peristomal area, such as within one or more first radial distances, e.g. 1cm, 2cm, and 3 cm, from the edge of the stoma. In other words, the first region may be seen as an inner area of the peristomal area.

One or more second secondary ostomy parameters may be indicative of the area, degree, or number of discoloured pixels within a second region of the peristomal area, such as outside one or more first radial distances, such as 1cm, 2cm, and 3 cm, from the edge of the stoma. In other words, the second region may be seen as an outer area of the peristomal area.

A second secondary ostomy parameter may be indicative of how many of the discoloured pixels in the peristomal area that are discoloured to a second degree or indicative of the area of pixels in the peristomal area that are discoloured to a second degree. For example, the second secondary ostomy parameter OP_2_2 may be based on the one or more stoma discoloration representations and may be given by $OP_ 2 _ 2 = N_DIS_ 2 / N_TOT ,$ where N_DIS_2 is the number of discoloured pixels in the peristomal area that are discoloured to a second degree, e.g. in a second range such as between 0.25 and 0.5, and N_TOT is the total number of discoloured pixels in the peristomal area.

A second tertiary ostomy parameter may be indicative of how many of the discoloured pixels in the peristomal area that are discoloured to a third degree or indicative of the area of pixels in the peristomal area that are discoloured to a third degree. For example, the second tertiary ostomy parameter OP_2_3 may be based on the one or more stoma discoloration representations and may be given by $OP_ 2 _ 3 = N_DIS_ 3 / N_TOT ,$ where N_DIS_3 is the number of discoloured pixels in the peristomal area that are discoloured to a third degree, e.g. in a third range such as between 0.5 and 0.75, and N_TOT is the total number of discoloured pixels in the peristomal area.

A second quaternary ostomy parameter may be indicative of how many of the discoloured pixels in the peristomal area that are discoloured to a fourth degree or indicative of the area of pixels in the peristomal area that are discoloured to a fourth degree. For example, the second quaternary ostomy parameter OP_2_4 may be based on the one or more stoma discoloration representations and may be given by $OP_ 2 _ 4 = N_DIS_ 4 / N_TOT ,$ where N_DIS_4 is the number of discoloured pixels in the peristomal area that are discoloured to a fourth degree, e.g. in a fourth range such as between 0.75 and 1 or larger than a threshold, and N_TOT is the total number of discoloured pixels in the peristomal area.

The first ostomy parameter may be a leakage parameter indicative of output distribution on the adhesive surface of the ostomy appliance. The second ostomy parameter or second set of second ostomy parameters may be leakage parameter(s) indicative of output distribution on the adhesive surface of the ostomy appliance.

The first ostomy parameter may be indicative of how much of the adhesive surface of the ostomy appliance that is discoloured. For example, the first ostomy parameter OP_1 may be based on the one or more appliance discoloration representations and may be given by $OP _ 1 = N _ TOT / N _ AA ,$

Where N_TOT is the total number of discoloured pixels in the adhesive surface area and N_AA is the total number of pixels in the adhesive surface area.

The first ostomy parameter may be indicative of a discoloured area of the adhesive surface of the ostomy appliance.

For example, the first ostomy parameter OP_1 may be based on the one or more appliance discoloration representations and may be given by $OP _ 1 = APP * N _ TOT$

Where N_TOT is the total number of discoloured pixels in the adhesive surface of the ostomy appliance and APP is an area per pixel.

The area of a pixel APP may be given as: $APP = HPP * WPP ,$ wherein HPP is the height of a pixel and WPP is the width of a pixel.

The height of a pixel HPP may be based on one or more of the image representations, such as a stoma background image representation and/or an appliance background image representation.

The height of a pixel, HPP may be given as: $DPP = AH / PH ,$ wherein AH is a height of the appliance (e.g. retrieved from a database) and PH is a pixel height of the appliance optionally determined as a number of pixels between two edges of the appliance counted along a vertical axis, e.g. in the appliance background image representation.

The width per pixel, WPP may be given as $WPP = AW / PW ,$ wherein AW is a width of the appliance (e.g. retrieved from a database) and PW is a pixel width of the appliance optionally determined as the number of pixels between two edges of the appliance counted along a horizontal axis, e.g. in the appliance background image representation.

The method may comprise determining one or more boundary lines based on the one or more image representations. The method may comprise determining a first boundary line, based on the one or more image representations, and wherein an ostomy representation comprises or is based on the first boundary line.

In one or more exemplary methods, the first boundary line may be indicative of a circumference or edge of a stomal area. The first boundary line may be indicative of a circumference or outer edge of an adhesive surface of the ostomy appliance.

The method may comprise determining a second boundary line, based on the one or more image representations, and wherein an ostomy representation comprises or is based on the second boundary line.

In one or more exemplary methods, the second boundary line may be indicative of a circumference or edge of the stoma. The second boundary line may be indicative of a circumference or inner edge of an adhesive surface of the ostomy appliance.

The method may comprise determining a third boundary line, based on the one or more image representations, and wherein an ostomy representation comprises or is based on the third boundary line.

In one or more exemplary methods, the third boundary line may be indicative of a boundary between a normal skin area of the peristomal area (non-discoloured) and a discoloured area of the peristomal area. The third boundary line may be indicative of a circumference or inner edge of an adhesive surface of the ostomy appliance.

The method may comprise determining a fourth boundary line e.g. based on the one or more image representations. Ostomy representation(s) and/or ostomy parameter(s), such as first and/or second ostomy parameter(s), may be based on the fourth boundary line. In one or more exemplary methods, the fourth boundary line may be indicative of a circumference of an output leakage on the adhesive surface of an ostomy appliance.

The first ostomy representation may comprise one or more boundary lines, such as the first boundary line and/or the second boundary line. The first ostomy representation may comprise the first ostomy parameter and/or one or more of the second ostomy parameters. The first ostomy representation may comprise the fourth boundary line.

Determining the one or more ostomy representations based on the image data may comprise determining a second ostomy representation based on the image data and/or transformed image data, e.g. the stoma image data and/or the appliance image data. The second ostomy representation, also denoted appliance representation, may be indicative of output distribution on the adhesive surface of the ostomy appliance. The second ostomy representation may comprise or be overlaid on the appliance image data or transformed appliance image data. The second ostomy representation may comprise one or more boundary lines, such as the first boundary line and/or the second boundary line. The second ostomy representation may comprise the third boundary line and/or the fourth boundary line.

Accordingly, determining one or more ostomy representations based on the image data may comprise determining boundary lines, e.g. of a first ostomy representation and/or of a second ostomy representation.

Determining one or more ostomy representations based on the image data may comprise determining a second ostomy parameter or a set of second ostomy parameters based on the image data and/or transformed image data, such as based on the stoma image data and/or the appliance image data.

The method optionally comprises determining one or more third ostomy parameters based on the one or more image representations. The method optionally comprises outputting the one or more third ostomy parameters.

A third primary ostomy parameter may be indicative of a shortest distance of a leakage of output to an edge of the ostomy appliance. The third ostomy parameter may be based on a first boundary line indicative of a circumference or outer edge of an adhesive surface of the ostomy appliance and a fourth boundary line indicative of a circumference of an output leakage on the adhesive surface of an ostomy appliance. The third ostomy parameter may be determined as a shortest (radial) distance between the first boundary line and the fourth boundary line. An angle may be associated with the third ostomy parameter, e.g. to indicate the direction in which the third ostomy parameter was measured or identified. The angle may be used for determining conversion parameter(s) for conversion between a pixel length and an absolute length.

In one or more exemplary methods, transforming the image data comprises determining a position parameter representative of a position of a camera image plane in relation to the stomal area and/or the adhesive surface, and wherein the transformed image data are based on the position parameter.

In one or more exemplary methods, the position parameter comprises an angle parameter representative of an angle between an optical axis of a camera being the source of the image data and an axial direction of the stomal area/normal to the adhesive surface. The transformed image data may be based on the angle parameter. In other words, transforming the image data may comprise determining an angle parameter representative of an angle between an optical axis of a camera being the source of the image data and an axial direction of the stomal area/normal to the adhesive surface. Thereby is enabled to compensate for image data that are not taken with the optical axis perpendicular to the adhesive surface of the ostomy appliance or perpendicular to the skin surface of the ostomist. Determining an angle parameter may comprise fitting the image data to a stomal area model image and/or an appliance model image and determining the angle parameter based on an image transformation providing a satisfactory fit of the image data to the stomal area model image and/or an appliance model.

In one or more exemplary methods, the position parameter comprises a distance parameter representative of a distance between a camera being the source of the image data and the stomal area/adhesive surface. The transformed image data may be based on the distance parameter. In other words, transforming the image data may comprise determining a distance parameter representative of a distance between a camera being the source of the image data and the stomal area/adhesive surface. A distance parameter allows for a (more precise) determination of a size of the stomal area/adhesive surface. Determining a distance parameter may comprise fitting the image data to a stomal area model image and/or an appliance model image and determining the distance parameter based on an image transformation providing a satisfactory fit of the image data to the stomal area model image and/or an appliance model.

In one or more exemplary methods, the position parameter comprises a rotation parameter representative of a rotational angle between an image axis of the image data and a reference axis of the stomal area/adhesive surface. The transformed image data may be based on the rotation parameter. The reference axis may be a vertical reference axis or a horizontal axis. In other words, transforming the image data may comprise determining a rotation parameter representative of a rotational angle between an image axis of the image data and a reference axis of the stomal area/adhesive surface. A rotation parameter allows to compensate for image data that are rotated, e.g. for a (more precise) determination of a directional ostomy condition. Determining a rotation parameter may comprise fitting the image data to a stomal area model image and/or an appliance model image and determining the rotation parameter based on an image transformation providing a satisfactory fit of the image data to the stomal area model image and/or an appliance model

In one or more exemplary methods, transforming the image data comprises applying a geometric transformation to the image data. The geometric transformation may be based on the position parameter, e.g. one or more of angle parameter, distance parameter, and rotation parameter.

In one or more exemplary methods, transforming the image data comprises fitting the image data to a stomal area model image and/or an appliance model image. For example, transforming the image data may comprise fitting stoma image data to a stomal area model and/or fitting appliance image data to an appliance model image.

In one or more exemplary methods, transforming the image data comprises identifying a first stoma reference indicator on the stomal area. The transformed image data may be based on the first stoma reference indicator. The first stoma reference indicator may be a perimeter of the stoma or other parameters relating to the stoma, e.g. a center of the stoma. Transforming the image data may comprise identifying a second stoma reference indicator and/or a third stoma reference indicator on the stomal area. The transformed image data may be based on the second stoma reference indicator and/or the third stoma reference indicator, e.g. on and/or outside the stomal area. The second stoma reference indicator may be a scar or other body mark, such as a birthmark, belly button, etc. The third stoma reference indicator may be a scar or other body mark, such as a birthmark, belly button, etc. A stoma reference indicator may be indicative of a position and/or a direction of the stoma reference indicator.

In one or more exemplary methods, transforming the image data comprises identifying a first appliance reference indicator on the adhesive surface of the ostomy appliance. The transformed image data may be based on the first appliance reference indicator. The first appliance reference indicator may be a perimeter of the ostomy appliance (or a part thereof), a center of the stomal opening of the ostomy appliance, or a perimeter of the stomal opening of the ostomy appliance. An appliance reference indicator may be indicative of a position and/or a direction of the appliance reference indicator.

In one or more exemplary methods, transforming the image data comprises identifying a second appliance reference indicator on the adhesive surface of the ostomy appliance. The second appliance reference indicator may be different from the first appliance reference indicator and is optionally a perimeter of the ostomy appliance (or a part thereof), a center of the stomal opening of the ostomy appliance, or a perimeter or edge of the stomal opening of the ostomy appliance. The transformed image data may be based on the second appliance reference indicator.

In one or more exemplary methods, transforming the image data comprises scaling, such as downscaling, the image data to a predetermined pixel size, such as NxM pixels, where N may be in the range from 100 to 2,500, e.g. in the range from 100 to 1,000, such as 256 or 512, and where M may be in the range from 100 to 2,500, e.g. in the range from 100 to 1,000, such as 256 or 512. M may be different from N.

In one or more exemplary methods, transforming the image data comprises centering the image data about a center or center region of the image data. In one or more exemplary methods, transforming the image data comprises identifying and selecting a stoma region (stoma region data) of the image data and/or an appliance region (appliance region data) of the image data, and optionally transforming the stoma region and/or the appliance region for provision of respective transformed stoma image data and/or transformed appliance image data. Selecting a stoma region and/or an appliance region may comprise cutting out the stoma region and/or the appliance region from respective stoma image data and/or appliance image data. The stoma region comprises the stomal area, i.e. the stoma and the peristomal area.

In one or more exemplary methods, transforming the image data comprises identifying and selecting a stoma region, centering the stoma region, optionally rotating the stoma region (e.g. using a geometric transformation based on rotation parameter), and downscaling the rotated stoma region for provision of transformed stoma image data.

In one or more exemplary methods, transforming the image data comprises identifying and selecting an appliance region, centering the appliance region, optionally rotating the appliance region (e.g. using a geometric transformation based on rotation parameter), and downscaling the rotated appliance region for provision of transformed appliance image data.

In one or more exemplary methods, scaling the image data comprises determining a scaling parameter. The transformed image data may be based on the scaling parameter. In one or more exemplary methods, determining one or more ostomy representations may comprise upscaling a representation, such as a first representation and/or a second representation or parts thereof, based on the scaling parameter.

In one or more exemplary methods, transforming the image data comprises applying an image conversion to the image data, such as the stoma image data. The image conversion may be based on one or more color channels including the red channel R and optionally the blue channel and/or the green channel of the image being converted. The converted image I_C may be given as: $I _ C = Abs \left(R-Average\left(G-B\right),\right.$ where R is the red channel in the image, G is the green channel, B is the blue channel. In other words, the red, blue, and green channels of the image may be converted into a single combined channel also denoted CC for each pixel of the image.

In one or more exemplary methods, obtaining image data comprises:
detecting, with an accessory device, a user input indicative of a request for image capture;
determining a position of the accessory device in relation to the adhesive surface of the ostomy appliance or in relation to the stomal area;
determining if the position of the accessory device in relation to the adhesive surface of the ostomy appliance or in relation to the stomal area satisfies image capture criteria; and
in accordance with a determination that the position of the accessory device in relation to the base plate of the ostomy appliance or in relation to the stomal area meets the image capture criteria,
   capturing image data of the ostomy appliance or the stomal area; and
   storing and/or transmitting the image data.

In one or more exemplary methods, obtaining image data comprises:
in accordance with a determination that the position of the accessory device in relation to the adhesive surface of the ostomy appliance or in relation to stomal area does not meet the image capture criteria, providing feedback to the user, the feedback being indicative of erroneous position of the accessory device.

Providing feedback to the user may comprise displaying a user interface element on a display of the accessory device. Providing feedback to the user may comprise determining a property of the user interface element based on the position of the accessory device in relation to the adhesive surface of the ostomy appliance.

In one or more exemplary methods, the method comprises, after providing feedback to the user:
determining a position of the accessory device in relation to the adhesive surface of the ostomy appliance;
determining if the position of the accessory device in relation to the adhesive surface of the ostomy appliance satisfies image capture criteria; and
in accordance with a determination that the position of the accessory device in relation to the adhesive surface of the ostomy appliance meets the image capture criteria,
   capturing image data of the ostomy appliance; and
   storing and/or transmitting the image data.

In one or more exemplary methods, determining a position of the accessory device in relation to the adhesive surface of the ostomy appliance comprises determining an angle between an optical axis of the camera and a proximal surface of the base plate.

In one or more exemplary methods, determining a position of the accessory device in relation to the stomal area comprises determining an angle between an optical axis of the camera and a reference surface of the stomal area, the reference surface being perpendicular to the axial direction.

In one or more exemplary methods, determining a position of the accessory device in relation to the adhesive surface of the ostomy appliance comprises determining a distance between the accessory device and the adhesive surface.

In one or more exemplary methods, the method comprises obtaining an ostomy appliance configuration, e.g. including an ostomy appliance identifier, and wherein determining a position of the accessory device in relation to the adhesive surface of the ostomy appliance is based on the ostomy appliance configuration.

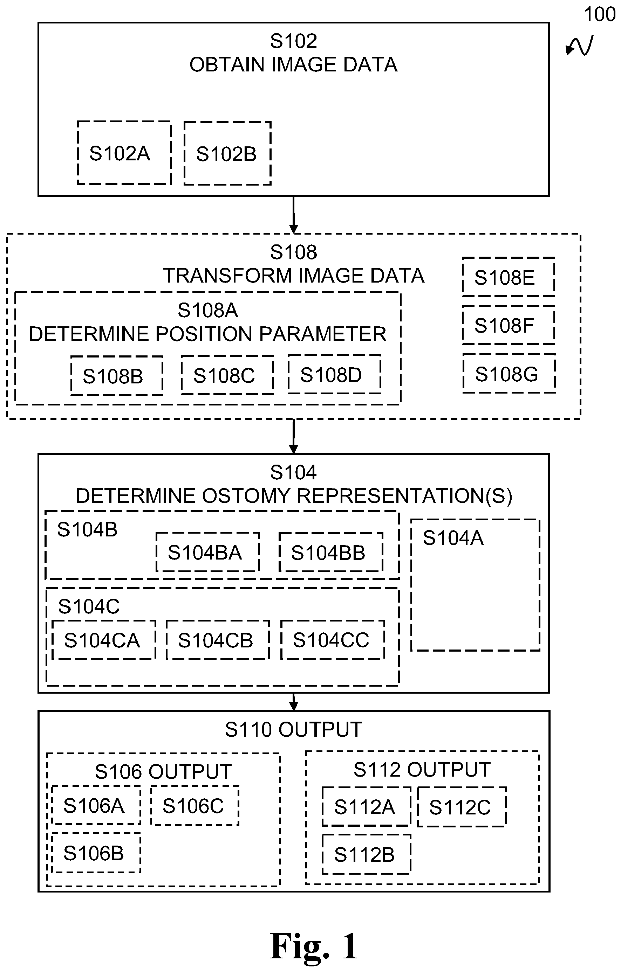

Fig. 1 shows a flow chart of an exemplary method for classifying an ostomy condition. The method 100 comprises obtaining 2 image data, e.g. with an accessory device. The image data, ID, comprises stoma image data, SID, of a stomal area including a stoma and/or appliance image data, AID, of an adhesive surface of an ostomy appliance. Thus, the method 100 optionally comprises obtaining 102A stoma image data and/or obtaining 102B appliance image data. The method 100 comprises determining S104 one or more ostomy representations, such as a first ostomy representation, including a first ostomy parameter based on the image data, e.g. based on SID and/or AID; and optionally outputting S106 the first ostomy parameter. The method 100 optionally comprises transforming S108 the image data, and determining S104 the one or more ostomy representations based on the image data comprises determining S104A the first ostomy parameter based on the transformed image data (transformed appliance image data AID_T and/or transformed stoma image data SID_T).

Determining S104 one or more ostomy representations optionally comprises determining S104B one or more image representations based on the image data or transformed image data; and determining S104C one or more ostomy representations including a first ostomy parameter based on the one or more image representations.

Determining S104B one or more image representations based on the image data or transformed image data optionally comprises determining S104BA one or more stoma image representations indicative of the stomal area and optionally determining S104BB one or more appliance image representations indicative of the adhesive surface of the ostomy appliance.

In one or more exemplary methods, the one or more stoma image representations comprises at least four stoma image representations including a first stoma image representation SIR_1, a second stoma image representation SIR_2, a third stoma image representation SIR_3, and a fourth stoma image representation SIR_4.

The first stoma image representation may be a stoma background image representation indicative of a background of the stoma image data, i.e. which part(s)/pixels of the stoma image data that are regarded or identified as background, i.e. outside the area covered by the adhesive surface (e.g. including part of user skin not covered by adhesive surface of ostomy appliance).

The second stoma image representation may be a stoma image representation indicative of the stoma, i.e. which part(s)/pixels of the ostomy image data that are regarded or identified as the stoma.

The third stoma image representation may be a normal skin image representation indicative of the normal skin of the peristomal area, i.e. which part(s)/pixels of the ostomy image data that are regarded or identified as not having discoloration.

The fourth stoma image representation may be a first discoloration representation indicative of a discoloration of the peristomal area, i.e. which part(s)/pixels of the ostomy image data that are regarded or identified as the peristomal area and discoloured.

In one or more exemplary methods, the one or more appliance image representations comprises at least three or at least four appliance image representations including a first appliance image representation AIR_1, optionally a second appliance image representation AIR_2, a third appliance image representation AIR_3, and a fourth appliance image representation AIR_4.

The first appliance image representation may be an appliance background image representation indicative of a background of the appliance image data, i.e. which part(s)/pixels of the appliance image data/transformed appliance image data that are regarded or identified as background (e.g. image part(s)/pixel(s) outside the area of the adhesive surface of the ostomy appliance).

The second appliance image representation may be a stomal opening image representation indicative of the stomal opening, i.e. which part(s)/pixels of the appliance image data/transformed appliance image data that are regarded or identified as the stomal opening.

The third appliance image representation may be an appliance area representation indicative of no appliance discoloration on the adhesive surface, i.e. no leak of output and thus which part(s)/pixels of the appliance image data/transformed appliance image data that are regarded or identified as the adhesive surface and not being discoloured by output.

The fourth appliance image representation may be a first appliance discoloration representation indicative of a discoloration of the adhesive surface of the ostomy appliance, i.e. which part(s)/pixels of the appliance image data that are regarded or identified as the adhesive surface and have a discoloration (leak of output).

Determining S104C one or more ostomy representations including a first ostomy parameter based on the one or more image representations optionally comprises determining S104CA a first ostomy representation OR_1 comprising the first ostomy parameter OP_1 based on one or more stoma image representations.

In one or more exemplary methods, the first ostomy representation OR_1 may be based on the first stoma image representation SIR_1, the second stoma image representation SIR_2, the third stoma image representation SIR_3, and the fourth stoma image representation SIR_4. The first ostomy representation OR_1 may comprise or be based on the ostomy image data/transformed ostomy image data.

Determining S104CA the first ostomy representation optionally comprises determining a second ostomy parameter OP_2 or a set of second ostomy parameters. In other words, the first ostomy representation OR_1 may comprise OP_1, and one or more second ostomy parameters.

Determining S104C one or more ostomy representations including a first ostomy parameter based on the one or more image representations optionally comprises determining S104CB a second ostomy representation OR_2 based on one or more appliance image representations.

In one or more exemplary methods, the second ostomy representation OR_2 may be based on the first appliance image representation, optionally the second appliance image representation, the third appliance image representation, and the fourth appliance image representation.

Outputting S106 the first ostomy parameter may comprise storing S106A the first ostomy parameter or a first ostomy representation comprising the first ostomy parameter in a memory and/or transmitting S106B the first ostomy parameter or a first ostomy representation comprising the first ostomy parameter to an accessory device and/or server device. Outputting S106 the first ostomy parameter may comprise displaying S106C the first ostomy parameter or a first ostomy representation comprising the first ostomy parameter on a display of an accessory device. Thereby a user and/or professional caretaker is able to verify and act on an ostomy condition in substantially real-time. For example, a user is able to or can be prompted to take measures to reduce the effects of an ostomy condition when changing the ostomy appliance, e.g. during a changing procedure in substantially real-time.

The method 100 comprises outputting 110 one or more ostomy representations, e.g. including the first ostomy representation OR_1 and/or the second ostomy representation OR_2. Outputting 110 one of rmore ostomy representations may comprise outputting 112 a second ostomy representation. Outputting S112 the second ostomy representation may comprise storing S112A the second ostomy representation in a memory and/or transmitting S112B the second ostomy representation to an accessory device and/or server device. Outputting S112 the second ostomy representation may comprise displaying S112C the second ostomy representation on a display of an accessory device. Thereby a user and/or professional caretaker is able to verify and act on an ostomy condition in substantially real-time. For example, a user is able to or can be prompted to take measures to reduce the effects of an ostomy condition when changing the ostomy appliance, e.g. during a changing procedure in substantially real-time.

Transforming S108 the image data comprises determining S108A a position parameter representative of a position of a camera image plane in relation to the stomal area and/or the adhesive surface, and wherein the transformed image data are based on the position parameter.

The position parameter optionally comprises an angle parameter representative of an angle between an optical axis of a camera being the source of the image data and an axial direction of the stomal area/normal to the adhesive surface and wherein the transformed image data are based on the angle parameter. Thus, determining S108A a position parameter may comprise determining 108B an angle parameter representative of an angle between an optical axis of a camera being the source of the image data and an axial direction of the stomal area/normal to the adhesive surface, and wherein the transformed image data are based on the angle parameter.

The position parameter optionally comprises a distance parameter representative of a distance between a camera being the source of the image data and the stomal area/adhesive surface, and wherein the transformed image data are based on the distance parameter. Thus, determining S108A a position parameter may comprise determining 108C a distance parameter representative of a distance between a camera being the source of the image data and the stomal area/adhesive surface, and wherein the transformed image data are based on the distance parameter.

The position parameter optionally comprises a rotation parameter representative of a rotational angle between an image axis of the image data and a reference axis of the stomal area/adhesive surface. Thus, determining S108A a position parameter may comprise determining 108D a rotation parameter representative of a rotational angle between an image axis of the image data and a reference axis of the stomal area/adhesive surface, and wherein the transformed image data are based on the rotation parameter.

In method 100, transforming S108 the image data optionally comprises identifying S108E one or more reference indicators of the image data, e.g. a first stoma reference indicator and/or a second stoma reference indicator of the stoma image data and/or a first appliance reference indicator and/or a second appliance reference indicator of the appliance image data. The transformed image data are optionally based on the reference indicator(s).

In method 100, transforming S108 the image data optionally comprises scaling S108F the image data to a predetermined pixel size, e.g. to a pixel size of 256x256 pixels. Scaling the image data comprises determining a scaling parameter, and wherein the transformed image data are based on the scaling parameter.

In method 100, transforming S108 the image data optionally comprises centering 108G the image data about a center or center region of the image data, e.g. based on a reference indicator (position and/or direction) of respective stoma image data and/or appliance image data. For example, transforming stoma image data may comprise centering the stoma image data about a center, perimeter, or center region of the stoma (e.g. identified as a first or second stoma reference identifier in S108E). In one or more exemplary methods, transforming appliance image data may comprise centering the appliance image data about a center, perimeter, or center region of the stomal opening of the ostomy appliance/baseplate and/or about a perimeter of the adhesive surface/baseplate of the ostomy appliance (e.g. identified as a first or second appliance reference identifier in S108E).

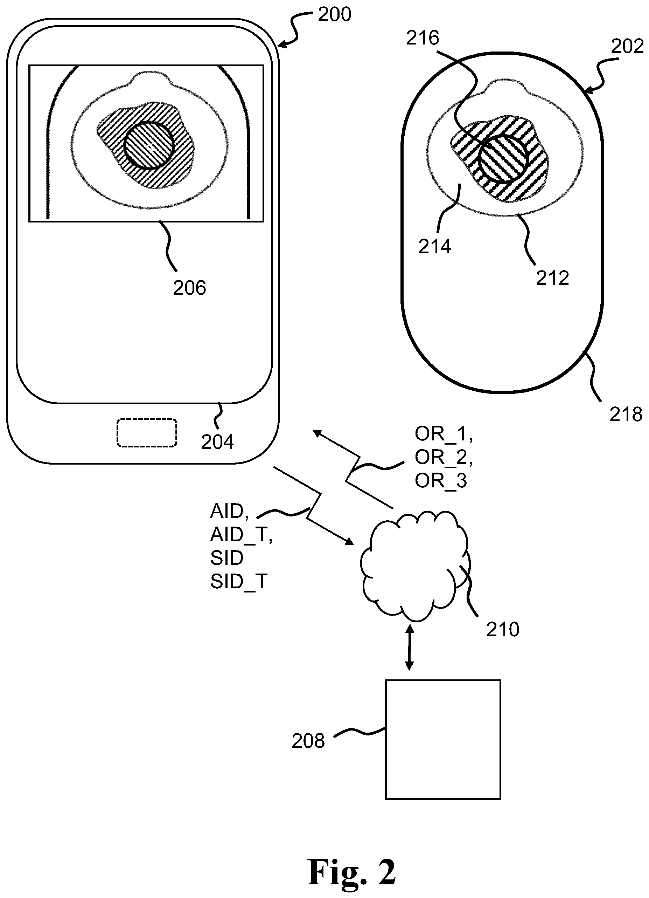

Fig. 2 shows an accessory device 200 and an ostomy appliance 202. The accessory device 200 is embodied as a smartphone and comprises a display 204 displaying an appliance image 206 representing appliance image data obtained by a camera of the accessory device 200. The accessory device 200 transmits the appliance image data AID and/or transformed appliance image data AID_T to server device 208 via network 210. The server device 210 determines one or more ostomy representations including one or more of first ostomy representation OR_1, second ostomy representation OR_2, and third ostomy representation OR_3, and outputs ostomy representation(s) by transmitting ostomy representation(s) to the accessory device 200. The accessory device 200 determines ostomy representation(s) by receiving, with the accessory device 200, the one or more ostomy representations from server device 208 and outputs ostomy representation(s) by displaying the first ostomy parameter or a first ostomy representation comprising the first ostomy parameter on the display 204 (not shown in Fig. 2). The ostomy appliance 202 comprises a baseplate 212 with adhesive surface 214 and stomal opening 216. An ostomy bag 218 is attached to the baseplate 212 for collection of output. During use, output 220 may leak between the adhesive surface and the skin surface of the user. Such output leakage may irritate and damage the skin due to the highly aggressive behaviour of the output. The present disclosure provides fast, uniform and reliable analysis and communication of such a leakage or ostomy condition based on the appliance image data.

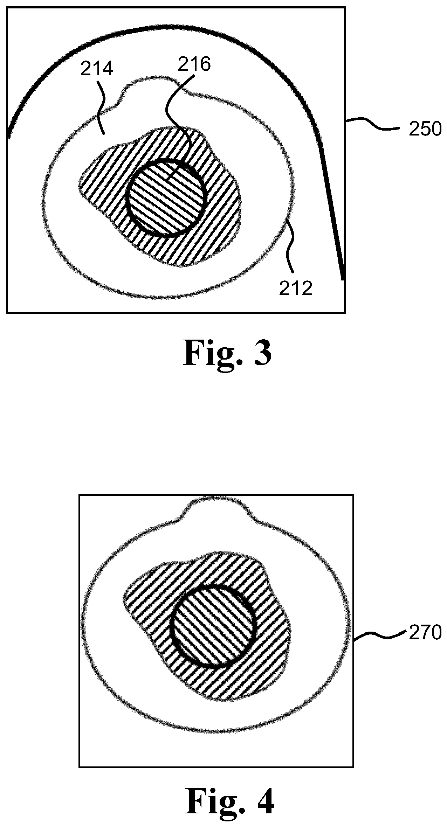

Fig. 3 shows an appliance image 250 (of ostomy appliance 202) representative of exemplary appliance image data captured with a camera of the accessory device 200. As can be seen, the user taking the appliance image 250 have rotated the camera slightly counter-clockwards and have not been able to center the stomal opening of the ostomy appliance.

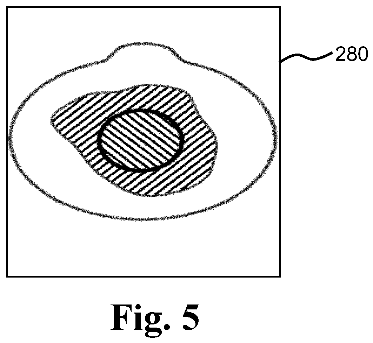

Fig. 4 shows an appliance image 270 representative of exemplary transformed appliance image data based on the appliance image data of Fig. 3 or Fig. 5. The appliance image data of appliance image 250 have been centered, rotated, e.g. by using geometric transformation, and downscaled to 256x256 pixels to provide transformed appliance data of appliance image 270.

Fig. 5 shows an appliance image 280 (of ostomy appliance 202) representative of exemplary appliance image data captured with a camera of the accessory device 200. As can be seen, the user taking the appliance image 280 have tilted the camera slightly such that the optical axis of the camera and a normal to the adhesive surface are slightly angled/not parallel. In this case, the appliance image data of appliance image 280 have been transformed by applying a geometric transformation based on an angle parameter, and downscaled to 256x256 pixels to provide transformed appliance data of image 270.

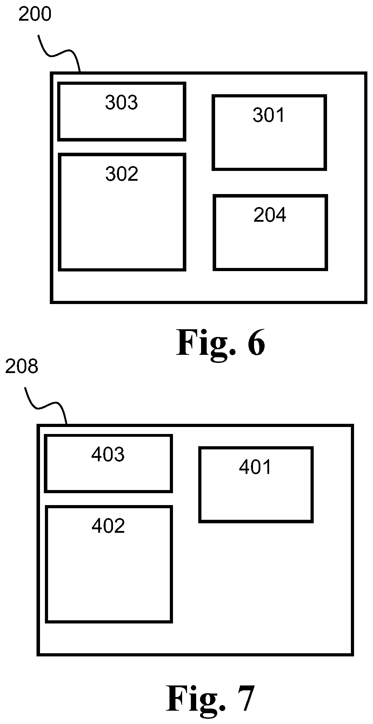

Fig. 6 is a block diagram illustrating an exemplary accessory device 200 according to this disclosure. The present disclosure relates to an accessory device 200 of an ostomy system. The accessory device comprises a display 204, memory module 301, a processor module 302, and a wireless interface 303. The accessory device 200 is configured to perform any of the methods disclosed herein, such as any of the methods shown in Fig. 1. The processor module 302 may be configured to perform any or at least some of the steps S102, S102A, S102B, S104, S104A, S104B, S104BA, S104BB, S104C, S104CA, S104CB, S104CC, S106, S106A, S106B, S106C, S108, S108A, S108B, S108C, S108D, S108E, S108F, S108G, S110, S112, S112A, S112B, S112C, see Fig. 1 and related description.

The operations of the accessory device 200 may be embodied in the form of executable logic routines (e.g., lines of code, software programs, etc.) that are stored on a non-transitory computer readable medium (e.g., the memory module 301) and are executed by the processor module 302. Furthermore, the operations of the accessory device 200 may be considered a method that the accessory device 200 is configured to carry out. Also, while the described functions and operations may be implemented in software, such functionality may as well be carried out via dedicated hardware or firmware, or some combination of hardware, firmware and/or software.

The memory module 301 may be one or more of a buffer, a flash memory, a hard drive, a removable media, a volatile memory, a non-volatile memory, a random access memory (RAM), or other suitable device. In a typical arrangement, the memory module 301 may include a non-volatile memory for long term data storage and a volatile memory that functions as system memory for the processor module 302. The memory module 301 may exchange data with the processor module 302 over a data bus. Control lines and an address bus between the memory module 301 and the processor module 302 also may be present (not shown in Fig. 6). The memory module 301 is considered a non-transitory computer readable medium.

Fig. 7 is a block diagram illustrating an exemplary server device 208 according to this disclosure. The present disclosure relates to a server device 208 of an ostomy system. The server device comprises a memory module 401, a processor module 402, and an interface 403. The server device 208 is configured to perform any of the methods disclosed herein, such as any of the methods shown in Fig. 1. The processor module 402 may be configured to perform any or at least some of the steps S102, S102A, S102B, S104, S104A, S104B, S104BA, S104BB, S104C, S104CA, S104CB, S104CC, S106, S106A, S106B, S106C, S108, S108A, S108B, S108C, S108D, S108E, S108F, S108G, S110, S112, S112A, S112B, S112C, see Fig. 1 and related description.

The operations of the server device 208 may be embodied in the form of executable logic routines (e.g., lines of code, software programs, etc.) that are stored on a non-transitory computer readable medium (e.g., the memory module 401) and are executed by the processor module 402. Furthermore, the operations of the server device 208 may be considered a method that the server device 208 is configured to carry out. Also, while the described functions and operations may be implemented in software, such functionality may as well be carried out via dedicated hardware or firmware, or some combination of hardware, firmware and/or software.

The memory module 401 may be one or more of a buffer, a flash memory, a hard drive, a removable media, a volatile memory, a non-volatile memory, a random access memory (RAM), or other suitable device. In a typical arrangement, the memory module 401 may include a non-volatile memory for long term data storage and a volatile memory that functions as system memory for the processor module 402. The memory module 401 may exchange data with the processor module 402 over a data bus. Control lines and an address bus between the memory module 401 and the processor module 402 also may be present (not shown in Fig. 7). The memory module 401 is considered a non-transitory computer readable medium.

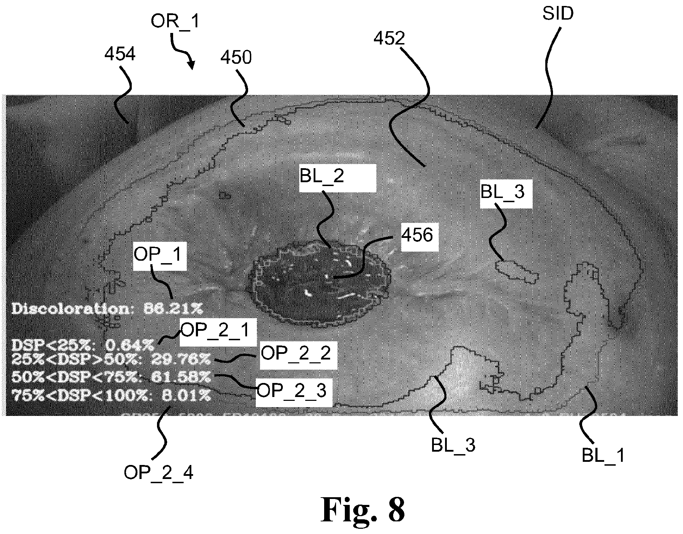

Fig. 8 illustrates an exemplary first ostomy representation OR_1. The first ostomy representation OR_1 comprises a first ostomy parameter OP_1 indicative of the indicative of discoloration of the stomal area (OP_1=86.21 % in the illustrated example). The first ostomy representation OR_1 comprises second ostomy parameters OP_2_1, OP_2_2, OP_2_3, and OP_2_4 indicative of discoloration severity percentage. The second primary ostomy parameter OP_2_1 (OP_2_1 = 0.64%) is indicative of the number of discoloured pixels with a first degree (DSP<25%) of discoloration out of the number of discoloured pixels. The second secondary ostomy parameter OP_2_2 (OP_2_2 = 29.76%) is indicative of the number of discoloured pixels with a second degree (25%<DSP<50%) of discoloration out of the number of discoloured pixels. The second tertiary ostomy parameter OP_2_3 (OP_2_1 = 61.58%) is indicative of the number of discoloured pixels with a third degree (50%<DSP<75%) of discoloration out of the number of discoloured pixels. The second quaternary ostomy parameter OP_2_4 (OP_2_4 = 8.01%) is indicative of the number of discoloured pixels with a fourth degree (75%<DSP<100%) of discoloration out of the number of discoloured pixels. The set of second ostomy parameters is determined based on the fourth stoma image representation.

The first ostomy representation OR_1 comprises first boundary line BL_1 (red line) indicative of a circumference or edge of the stomal area, e.g. indicative of a boundary between the normal skin area 450 and background 454 of the stoma image data. The first boundary line is based on the first stoma image representation and/or the third stoma image representation.

The first ostomy representation OR_1 comprises second boundary line BL_2 (green line) indicative of a circumference or edge of the stoma 456, wherein the second boundary line is based on the second stoma image representation and/or the fourth stoma image representation.

The first ostomy representation OR_1 comprises third boundary lines BL_3 (blue lines) indicative of a boundary between a normal skin area 450 of the peristomal area (non-discoloured) and a discoloured area 452 of the peristomal area. The third boundary lines BL_3 are based on the third stoma image representation and/or the fourth stoma image representation.

The first ostomy representation OR_1 comprises or is overlaid on the stoma image data SID on which the first ostomy representation OP_1 is based.

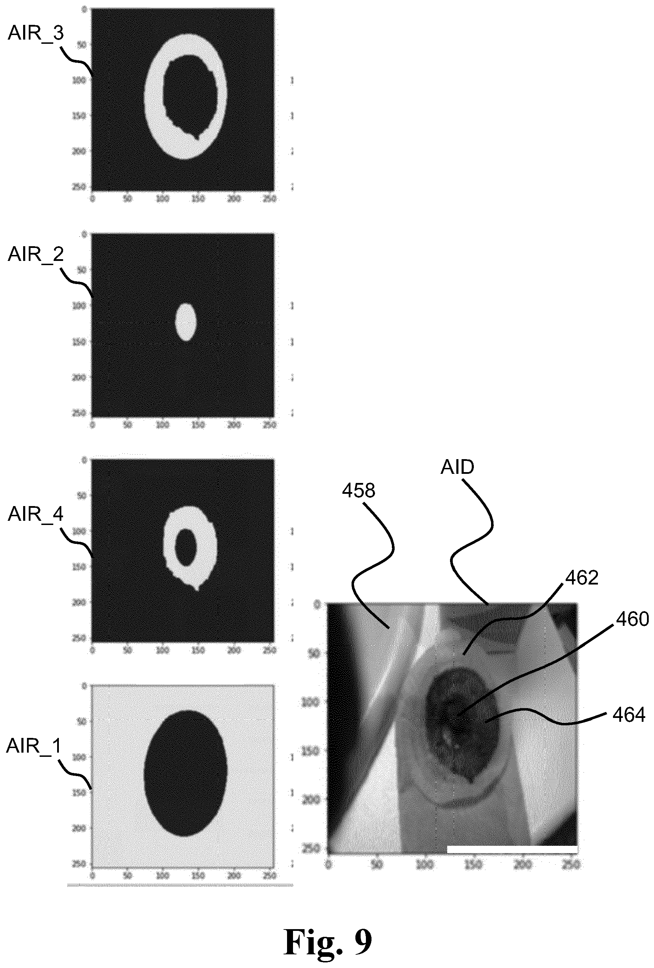

Fig. 9 shows exemplary appliance image representations with appliance image data AID forming the basis therefore. The first appliance image representation AIR_1 of 256x256 pixels being an appliance background image representation (binary mask) indicative of a background 458 of the appliance image data, i.e. which part(s)/pixels of the appliance image data/transformed appliance image data that are regarded or identified as background (e.g. image part(s)/pixel(s) outside the area of the adhesive surface of the ostomy appliance). Yellow represents the binary value 1 of the binary mask (i.e. pixel is part of background) and purple represents the binary value 0 (i.e. pixel is not part of background).

The second appliance image representation AIR_2 of 256x256 pixels is a stomal opening image representation indicative of the stomal opening 460, i.e. which part(s)/pixels of the appliance image data/transformed appliance image data that are regarded or identified as the stomal opening. Yellow represents the binary value 1 of the binary mask (i.e. pixel is part of stomal opening) and purple represents the binary value 0 (i.e. pixel is not part of stomal opening).

The third appliance image representation AIR_3 of 256x256 pixels is an appliance area representation indicative of no appliance discoloration (clean adhesive surface 462) on the adhesive surface of the ostomy appliance, i.e. no leak of output and thus which part(s)/pixels of the appliance image data/transformed appliance image data that are regarded or identified as the adhesive surface and not being discoloured by output. Yellow represents the binary value 1 of the binary mask (i.e. pixel is not discoloured) and purple represents the binary value 0 (i.e. pixel is not part of non-discoloured adhesive surface).

The fourth appliance image representation AIR_4 of 256x256 pixels is a first appliance discoloration representation indicative of a discoloration (discoloured adhesive surface 464) of the adhesive surface of the ostomy appliance, i.e. which part(s)/pixels of the appliance image data that are regarded or identified as the adhesive surface and have a discoloration (leak of output). Yellow represents the binary value 1 of the binary mask (i.e. pixel is discoloured) and purple represents the binary value 0 (i.e. pixel is not part of discoloured adhesive surface).

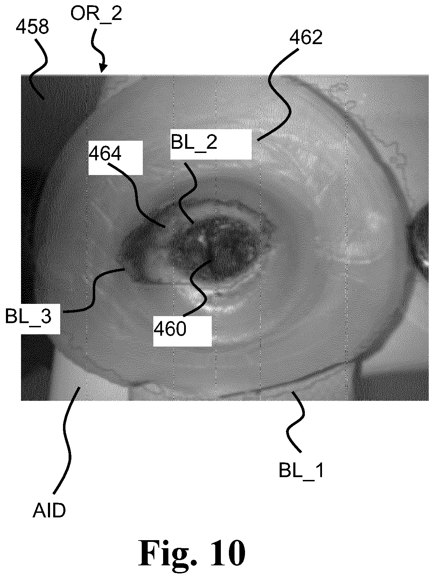

Fig. 10 shows an exemplary second ostomy representation OR_2 based on four appliance image representations as also described in relation to Fig. 9. The second ostomy representation OR_2 comprises a first boundary line BL_1 (red line) indicative of a circumference or edge of the adhesive surface of the ostomy appliance. The first boundary line BL_1 is based on the first appliance image representation and/or the third appliance image representation.

The second ostomy representation OR_2 comprises second boundary line BL_2 (green line) indicative of a circumference or edge of the stomal opening of the adhesive surface, wherein the second boundary line is based on the second appliance image representation and/or the fourth appliance image representation.

The second ostomy representation OR_2 comprises third boundary line BL_3 (blue line) indicative of a boundary between a discoloured part (output leak) and a non-discoloured part (clean) of the adhesive surface. The third boundary lines BL_3 are based on the third stoma image representation and/or the fourth stoma image representation.

The second ostomy representation OR_2 comprises or is overlaid on the appliance image data AID on which the second ostomy representation OP_2 is based.

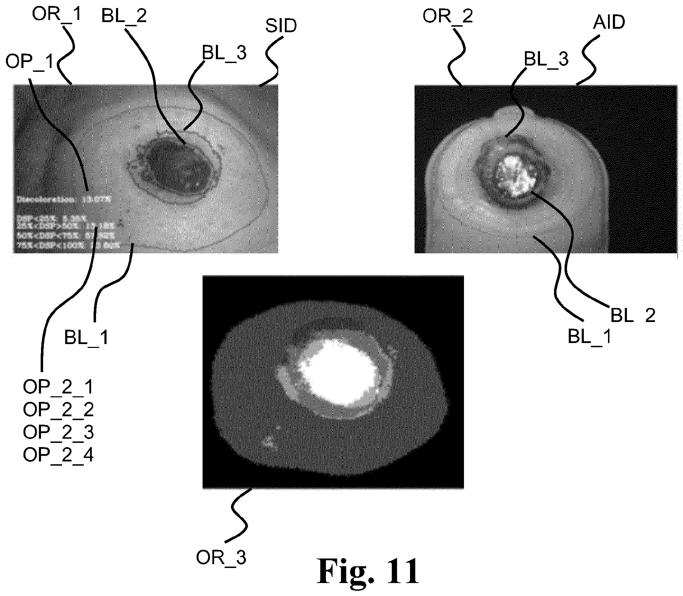

Fig. 11 shows exemplary ostomy representations OR_1, OR_2, and OR_3 for corresponding stoma image data SID and appliance image data AID. The third ostomy representation OR_3 is based on one or more of the stoma image representations and one or more appliance image representations. In the third ostomy representation, the blue part is indicative of the fourth appliance image representation and the light gray part is indicative of the discoloured part of the peristomal area, i.e. indicative of the third stoma image representation.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering.

Furthermore, the labelling of a first element does not imply the presence of a second element and vice versa.

It may be appreciated that Figs. 1-7 comprise some modules or operations which are illustrated with a solid line and some modules or operations which are illustrated with a dashed line. The modules or operations which are comprised in a solid line are modules or operations which are comprised in the broadest example embodiment. The modules or operations which are comprised in a dashed line are example embodiments which may be comprised in, or a part of, or are further modules or operations which may be taken in addition to the modules or operations of the solid line example embodiments. It should be appreciated that these operations need not be performed in order presented. Furthermore, it should be appreciated that not all of the operations need to be performed. The exemplary operations may be performed in any order and in any combination.

It is to be noted that the word "comprising" does not necessarily exclude the presence of other elements or steps than those listed.

It is to be noted that the words "a" or "an" preceding an element do not exclude the presence of a plurality of such elements.

It should further be noted that any reference signs do not limit the scope of the claims, that the exemplary embodiments may be implemented at least in part by means of both hardware and software, and that several "means", "units" or "devices" may be represented by the same item of hardware.

The various exemplary methods, devices, and systems described herein are described in the general context of method steps processes, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program modules may include routines, programs, objects, components, data structures, etc. that perform specified tasks or implement specific abstract data types. Computer-executable instructions, associated data structures, and program modules represent examples of program code for executing steps of the methods disclosed herein. The particular sequence of such executable instructions or associated data structures represents examples of corresponding acts for implementing the functions described in such steps or processes.

Although features have been shown and described, it will be understood that they are not intended to limit the claimed invention, and it will be made obvious to those skilled in the art that various changes and modifications may be made without departing from the scope of the claimed invention. The specification and drawings are, accordingly to be regarded in an illustrative rather than restrictive sense. The scope of the invention is solely defined by the appended claims.

### List of references

100 method for classifying an ostomy condition
S102 obtaining image data
S102A obtaining stoma image data
S102B obtaining appliance image data
S104 determining one or more ostomy representations
S104A determining the first ostomy parameter based on the transformed image data
S104B determining one or more image representations
S104BA determining one or more stoma image representations
S104BB determining one or more appliance image representations
S104C determining one or more ostomy representations including a first ostomy parameter based on the one or more image representations
S104CA determining a first ostomy representation
S104CB determining a second ostomy representation
S104CC determining a third ostomy representation
S106 outputting first ostomy representation/first ostomy parameter
S106A storing the first ostomy parameter or a first ostomy representation comprising the first ostomy parameter
S106B transmitting with server device and/or receiving with accessory device the first ostomy parameter or a first ostomy representation comprising the first ostomy parameter
S106C displaying the first ostomy parameter or a first ostomy representation comprising the first ostomy parameter
S108 transforming the image data
S108A determining a position parameter
S108B determining an angle parameter
S108C determining a distance parameter
S108D determining a rotation parameter
S108E identifying one or more reference indicators of the image data
S108F scaling the image data to a predetermined pixel size
S108G centering the image data
S110 outputting one or more ostomy representations
S112 outputting second ostomy representation
S112A storing the second ostomy representation
S112B transmitting with server device and/or receiving with accessory device the second ostomy representation
S112C displaying the second ostomy representation
200 accessory device
202 ostomy appliance
204 display
206 appliance image
208 server device
210 network
212 baseplate
214 adhesive surface
216 stomal opening
218 ostomy bag
220 output
250 appliance image of appliance image data
270 appliance image of transformed appliance image data
280 appliance image of appliance image data
301 memory module
302 processor module
302A image transformer
302B ostomy representation determiner
302C image representation determiner
303 wireless interface
401 memory module
402 processor module
402A image transformer
402B ostomy representation determiner
402C image representation determiner
403 interface
450 normal skin area
452 discoloured skin area
454 background
456 stoma
AID appliance image data
AID_T transformed appliance image data
AIR_1 first appliance image representation
AIR_2 second appliance image representation
AIR_3 third appliance image representation
AIR_4 fourth appliance image representation
BL_1 first boundary line
BL_2 second boundary line
BL_3 third boundary line
ID image data
ID_T transformed image data
IR_1 first image representation
IR_2 second image representation
IR_3 third image representation
IR_4 fourth image representation
OP_1 first ostomy parameter
OP_2_1 second primary ostomy parameter
OP_2_2 second secondary ostomy parameter
OP_2_3 second tertiary ostomy parameter
OP_2_4 second quaternary ostomy parameter
OR_1 first ostomy representation
OR_2 second ostomy representation
OR_3 third ostomy representation
SID stoma image data
SID_T transformed stoma image data
SIR_1 first stoma image representation
SIR_2 second stoma image representation
SIR_3 third stoma image representation
SIR_4 fourth stoma image representation

## Claims

1. A method for classifying an ostomy condition, the method comprising:
obtaining (S102) image data (ID), the image data comprising stoma image data (SID) of a stomal area including a stoma (456) and/or appliance image data (AID) of an adhesive surface of an ostomy appliance (202);
determining (S104) one or more ostomy representations including a first ostomy parameter (OP_1) based on the image data (ID); and
outputting (S110) the first ostomy parameter (OP_1);
wherein determining (S104) one or more ostomy representations including a first ostomy parameter (OP_1) based on the image data (ID) comprises:
transforming (S108) the image data (ID);
determining (S104B) one or more image representations based on the transformed image data (ID_T); and
determining (S104C) one or more ostomy representations including a first ostomy parameter (OP_1) based on the one or more image representations, **characterized in that**
the first ostomy parameter (OP_1) is a discoloration index indicative of discoloration of the stomal area or a leakage parameter indicative of output distribution on the adhesive surface.

2. Method according to claim 1, wherein the one or more ostomy representations further includes a first ostomy representation (OR_1), and wherein the method further comprises outputting the first ostomy representation (OR_1).

3. Method according to any of claims 1-2, wherein the one or more image representations is a binary mask.

4. Method according to any of claims 1-3, wherein the one or more image representations comprises a stoma background image representation and/or an appliance background image representation.

5. Method according to any of claims 1-4, wherein the one or more image representations comprises a stoma image representation.

6. Method according to any of claims 1-5, wherein the one or more image representations comprises a first stoma discoloration representation and/or a first appliance discoloration representation.

7. Method according to any of claims 1-6, wherein the one or more image representations comprises an appliance area representation.

8. Method according to any of claims 1-7, the method comprising determining one or more boundary lines based on the one or more image representations.

9. Method according to claim 8, wherein the one or more boundary lines includes a first boundary line (BL_1) indicative of an edge of the stomal area and/or indicative of an outer edge of the adhesive surface.

10. Method according to any of claims 8-9, wherein the one or more boundary lines includes a second boundary line (BL_1) indicative of an edge of the stoma (456) and/or indicative of an inner edge of the adhesive surface.

11. Method according to claim 8, wherein the one or more boundary lines include one or more of (i) a first boundary line (BL_1) indicative of an edge of the stomal area and/or an outer edge of the adhesive surface, (ii) a second boundary line (BL_2) indicative of an edge of the stoma and/or an inner edge of the adhesive surface, (iii) a third boundary line (BL_3) indicative of a boundary between a non-discoloured area (450) of the peristomal area and a discoloured area (452) of the peristomal area and/or of an inner edge of the adhesive surface, and (iv) a fourth boundary line indicative of a circumference of an output leakage on the adhesive surface.

12. Method according to any of claims 1-11, wherein transforming the image data comprises applying a geometric transformation to the image data.

13. Method according to any of claims 1-12, wherein transforming the image data comprises identifying a first stoma reference indicator on the stomal area and/or a first appliance reference indicator on the adhesive surface.

14. Method according to any of claims 1-13, wherein transforming the image data comprises applying an image conversion to the image data.

15. An accessory device (200) of an ostomy system, the accessory device comprising a display (204), a memory module (301), a processor module (302), and a wireless interface (303), the accessory device being configured to perform any of the methods according to claims 1-14.

## Patentansprüche

1. Verfahren zum Klassifizieren eines Stomazustands, wobei das Verfahren Folgendes umfasst:
Erlangen (S102) von Bilddaten (ID), wobei die Bilddaten Stomabilddaten (SID) eines Stomagebiets, einschließlich eines Stomas (456), und/oder Versorgungsbilddaten (AID) einer Klebemitteloberfläche einer Stomaversorgung (202) umfassen;
Bestimmen (S104) einer oder mehrerer Stomadarstellungen, einschließlich eines ersten Stomaparameters (OP_1), auf der Grundlage der Bilddaten (ID); und
Ausgeben (S110) des ersten Stomaparameters (OP_1);
wobei Bestimmen (S104) einer oder mehrerer Stomadarstellungen, einschließlich eines ersten Stomaparameters (OP_1), auf der Grundlage der Bilddaten (ID), Folgendes umfasst:
Transformieren (S108) der Bilddaten (ID);
Bestimmen (S104B) einer oder mehrerer Bilddarstellungen, auf der Grundlage der transformierten Bilddaten (ID_T); und
Bestimmen (S104C) einer oder mehrerer Stomadarstellungen, einschließlich eines ersten Stomaparameters (OP_1), auf der Grundlage der einen oder der mehreren Bilddarstellungen, **dadurch gekennzeichnet, dass**
der erste Stomaparameter (OP_1) ein Verfärbungsindex ist, der eine Verfärbung des Stomagebiets angibt, oder ein Leckageparameter, der eine Ausscheidungsverteilung auf der Klebemitteloberfläche angibt.

2. Verfahren nach Anspruch 1, wobei die eine oder die mehreren Stomadarstellungen ferner eine erste Stomadarstellung (OR_1) beinhalten und wobei das Verfahren ferner Ausgeben der ersten Stomadarstellung (OR_1) umfasst.

3. Verfahren nach einem der Ansprüche 1 - 2, wobei die eine oder die mehreren Bilddarstellungen eine Binärmaske sind.

4. Verfahren nach einem der Ansprüche 1 - 3, wobei die eine oder die mehreren Bilddarstellungen eine Stomahintergrundbilddarstellung und/oder eine Versorgungshintergrundbilddarstellung umfassen.

5. Verfahren nach einem der Ansprüche 1 - 4, wobei die eine oder die mehreren Bilddarstellungen eine Stomabilddarstellung umfassen.

6. Verfahren nach einem der Ansprüche 1 - 5, wobei die eine oder die mehreren Bilddarstellungen eine erste Stomaverfärbungsdarstellung und/oder eine erste Versorgungsverfärbungsdarstellung umfassen.

7. Verfahren nach einem der Ansprüche 1 - 6, wobei die eine oder die mehreren Bilddarstellungen eine Versorgungsgebietsdarstellung umfassen.

8. Verfahren nach einem der Ansprüche 1 - 7, wobei das Verfahren Bestimmen einer oder mehrerer Umgrenzungslinien auf der Grundlage der einen oder der mehreren Bilddarstellungen umfasst.

9. Verfahren nach Anspruch 8, wobei die eine oder die mehreren Umgrenzungslinien eine erste Umgrenzungslinie (BL_1), die einen Rand des Stomagebiets angibt und/oder einen Außenrand der Klebemitteloberfläche angibt, umfasst.

10. Verfahren nach einem der Ansprüche 8 - 9, wobei die eine oder die mehreren Umgrenzungslinien eine zweite Umgrenzungslinie (BL_1), die einen Rand des Stomas (456) angibt und/oder einen Innenrand der Klebemitteloberfläche angibt, umfasst.

11. Verfahren nach Anspruch 8, wobei die eine oder die mehreren Umgrenzungslinien eine oder mehrere beinhalten von (i) einer ersten Umgrenzungslinie (BL_1), die einen Rand des Stomagebiets und/oder einen Außenrand der Klebemitteloberfläche angibt, (ii) einer zweiten Umgrenzungslinie (BL_2), die einen Rand des Stomas und/oder einen Innenrand der Klebemitteloberfläche angibt, (iii) einer dritten Umgrenzungslinie (BL_3), die eine Grenze zwischen einem nicht-verfärbten Gebiet (450) des Peristomagebiets und einem verfärbten Gebiet (452) des Peristomagebiets und/oder von einem Innenrand der Klebemitteloberfläche angibt, und (iv) einer vierten Umgrenzungslinie, die eine Umfangslinie einer Ausscheidungsleckage auf der Klebemitteloberfläche angibt.

12. Verfahren nach einem der Ansprüche 1 - 11, wobei Transformieren der Bilddaten Anwenden einer geometrischen Transformation auf die Bilddaten umfasst.

13. Verfahren nach einem der Ansprüche 1 - 12, wobei Transformieren der Bilddaten Identifizieren eines ersten Stomareferenzindikators in dem Stomagebiet und/oder eines ersten Versorgungsreferenzindikators auf der Klebemitteloberfläche umfasst.

14. Verfahren nach einem der Ansprüche 1 - 13, wobei Transformieren der Bilddaten Anwenden einer Bildumwandlung auf die Bilddaten umfasst.

15. Zubehörvorrichtung (200) eines Stomasystems, wobei die Zubehörvorrichtung ein Display (204), ein Arbeitsspeichermodul (301), ein Prozessormodul (302) und eine drahtlose Schnittstelle (303) umfasst; wobei die Zubehörvorrichtung ausgelegt ist zum Durchführen der Verfahren nach Ansprüchen 1 - 14.

## Revendications

1. Procédé de classification d'un état de stomie, le procédé comprenant :
l'obtention (S102) de données d'image, les données d'image comprenant des données d'image de stomie (SID) d'une zone de stomie comportant une stomie (456) et/ou des données d'image d'appareil (AID) d'une surface adhésive d'un appareil de stomie (202) ;
la détermination (S104) d'une ou plusieurs représentations de stomie comportant un premier paramètre de stomie (OP_1) sur la base des données d'image ; et
la sortie (S110) du premier paramètre de stomie (OP_1) ; dans lequel la détermination (S104) d'une ou plusieurs représentations de stomie comportant un premier paramètre de stomie (OP_1) sur la base des données d'image (ID) comprend :
la transformation (S108) des données d'image (ID) ;
la détermination (S104B) d'une ou de plusieurs représentations d'image sur la base des données d'image transformées (ID_T) ; et
la détermination (S104C) d'une ou de plusieurs représentations de stomie comportant un premier paramètre de stomie (OP_1) sur la base des une ou plusieurs représentations d'image, **caractérisé en ce que**
le premier paramètre de stomie (OP_1) est un indice de décoloration indiquant une décoloration de la zone de stomie ou un paramètre de fuite indiquant une distribution de sortie sur la surface adhésive.

2. Procédé selon la revendication 1, dans lequel la ou les représentations de stomie comportent en outre une première représentation de stomie (OR_1), et dans lequel le procédé comprend en outre la sortie de la première représentation de stomie (OR_1).

3. Procédé selon l'une quelconque des revendications 1 et 2, dans lequel la ou les représentations d'image sont un masque binaire.

4. Procédé selon l'une quelconque des revendications 1 à 3, dans lequel la ou les représentations d'images comprennent une représentation d'image d'arrière-plan de stomie et/ou une représentation d'image d'arrière-plan d'appareil.

5. Procédé selon l'une quelconque des revendications 1 à 4, dans lequel la ou les représentations d'image comprennent une représentation d'image de stomie.

6. Procédé selon l'une quelconque des revendications 1 à 5, dans lequel la ou les représentations d'image comprennent une première représentation de décoloration de stomie et/ou une première représentation de décoloration d'appareil.

7. Procédé selon l'une quelconque des revendications 1 à 6, dans lequel la ou les représentations d'image comprennent une représentation de zone d'appareil.

8. Procédé selon l'une quelconque des revendications 1 à 7, le procédé comprenant la détermination d'une ou plusieurs limites sur la base de la ou des représentations d'image.

9. Procédé selon la revendication 8, dans lequel la ou les limites comportent une première limite (BL_1) indiquant un bord de la zone de stomie et/ou indiquant un bord externe de la surface adhésive.

10. Procédé selon l'une quelconque des revendications 8 à 9, dans lequel la ou les limites comportent une seconde limite (BL_1) indiquant un bord de la stomie (456) et/ou indiquant un bord interne de la surface adhésive.

11. Procédé selon la revendication 8, dans lequel la ou les limites comportent une ou plusieurs parmi (i) une première limite (BL_1) indiquant un bord de la zone de stomie et/ou un bord externe de la surface adhésive, (ii) une deuxième limite (BL_2) indiquant un bord de la stomie et/ou un bord interne de la surface adhésive, (iii) une troisième limite (BL_3) indiquant une limite entre une zone non décolorée (450) de la zone péristomale et une zone décolorée (452) de la zone péristomale et/ou d'un bord interne de la surface adhésive, et (iv) une quatrième limite indiquant une circonférence d'une fuite de sortie sur la surface adhésive.

12. Procédé selon l'une quelconque des revendications 1 à 11, dans lequel la transformation des données d'image comprend l'application d'une transformation géométrique aux données d'image.

13. Procédé selon l'une quelconque des revendications 1 à 12, dans lequel la transformation des données d'image comprend l'identification d'un premier indicateur de référence de stomie sur la zone de stomie et/ou d'un premier indicateur de référence d'appareil sur la surface adhésive.

14. Procédé selon l'une quelconque des revendications 1 à 13, dans lequel la transformation des données d'image comprend l'application d'une conversion d'image aux données d'image.

15. Dispositif accessoire (200) d'un système de stomie, le dispositif accessoire comprenant un afficheur (204), un module de mémoire (301), un module de processeur (302) et une interface sans fil (303), le dispositif accessoire étant configuré pour réaliser l'un quelconque des procédés selon les revendications 1 à 14.
